# EUROPEAN PATENT APPLICATION

(11) **EP 3 785 665 A1**
(43) Date of publication of application: **03.03.2021**
(21) Application number: 18915822.3
(22) Date of filing: 19.11.2018
(51) Int. Cl.: A61F 2/07

(54) **ASCENDING AORTIC STENT GRAFT**

(30) Priority: 25.04.2018 CN 201810379341; 25.04.2018 CN 201820600490 U
(71) Applicant: Shanghai Changhai Hospital, Shanghai 200433 (CN)
(72) Inventor: JING, Zaiping, Shanghai 200433 (CN); FENG, Jiaxuan, Shanghai 200433 (CN); BAO, Xianhao, Shanghai 200433 (CN); FENG, Rui, Shanghai 200433 (CN); ZHOU, Jian, Shanghai 200433 (CN); LU, Qingsheng, Shanghai 200433 (CN); LI, Tao, Shanghai 200433 (CN); BAO, Junmin, Shanghai 200433 (CN); ZHAO, Zhiqing, Shanghai 200433 (CN); MAO, Huajuan, Shanghai 200433 (CN); ZHUANG, Yufeng, Shanghai 200433 (CN)
(74) Representative: Haseltine Lake Kempner LLP
(86) International application number: PCT/CN2018/116151
(87) International publication number: WO 2019/205599

(57) **Abstract**

Disclosed herein is a stent graft for ascending aorta, comprising a stent; a cover attachment at least partially covering the stent; a valve leaflet for reconstructing aortic valves, which are positioned at the proximal end of the stent and attached to the cover attachment and the stent. The valve leaflet for reconstructing aortic valves is configured to allow blood to flow from the left ventricle to the aorta and prevent blood from flowing from the aorta back to the left ventricle. In addition, the stent graft has two windows aligning with two openings of the coronary artery. Therefore, for the aortic root diseases involving aortic sinus and aortic valves, the stent graft for ascending aorta can be implanted into ascending aortic root by minimally invasive endovascular surgery to repair the ascending aorta lesions and reconstruct the aortic valves, aortic sinus, openings of the coronary artery, thereby achieving overall cure.

## Description

### CROSS REFERENCE TO RELATED APPLICATIONS

This application claims priorities to Chinese Patent Application Serial No. 201810379341.2, filed on April 25, 2018, entitled "STENT GRAFT FOR ASCENDING AORTA", and Chinese Patent Application Serial No. 201820600490.2, filed on April 25, 2018, entitled "STENT GRAFT FOR ASCENDING AORTA", the entire disclosures of which are herein incorporated by reference.

### FIELD OF THE INVENTION

The present invention relates to a medical device, more particularly, to a stent graft for ascending aorta for treating ascending aortic dissection involving aortic sinus, coronary artery openings, aortic valve and aortic aneurysm, aortic valve stenosis or insufficiency with aortic aneurysm and the like.

### BACKGROUND OF THE INVENTION

Aortic dissection is one of the most serious diseases involving aorta. High-speed and high-pressure blood in the aorta flows into the outside of the tunica media or the junction of the tunica media and the tunica adventitia through the tearing opening of the tunica intima on the aortic wall, and thus the true and false cavities of the aorta are formed and expaned along the aortic axis, thereby forming aortic dissection and leading to aortic rupture or branch artery ischemia (e.g. myocardial infarction, cerebral infarction, internal organs or lower extremity ischemia), etc.. In previous studies, statistics has been performed on dissections in different parts of the aorta according to the location of the proximal tearing opening of the aortic dissection. Aortic dissection with proximal tearing opening in the ascending aorta accounts for 65% of all aortic dissections. Aortic dissection with proximal tearing opening in the aortic arch accounts for 5% of all aortic dissections. Aortic dissection with proximal tearing opening in the descending aorta accounts for about 30% of all aortic dissections. Aortic aneurysm refers to pathological expansion of the aortawhich exceeds 50% of the diameter of the normal blood vessel. It can occur in the aortic sinus, ascending aorta, aortic arch, descending thoracic aorta, thoracic-abdominal aorta, and abdominal aorta. Rupture of the aortic aneurysm can cause sudden death. The aortic aneurysm in the ascending aorta typically involves aortic sinus, which results in deformation of the aortic annulus and aortic valve insufficiency due to separation of the valve leaflet, thereby leading to heart failure due to long-term effects.

For the aortic dissection and aortic aneurysm in which the proximal tearing opening and the aortic aneurysms are in the descending aorta, aortic arch and the middle 1/3 of the ascending aorta, they can be treated by implanting a stent graft covered by an artificial vascular membrane to isolate the proximal tearing opening, thereby shrinking the false cavity and aneurysm due to thrombosis and expanding the true cavity of the dissection. Compared with conventional open surgery, this treatment does not need to perform thoracotomy, blood transfusion, and extracorporeal circulation, and thus it is simple, minimally invasive, and effective.

With the development of endovascular stent graft and technology, minimally invasive interventional surgery for treatment of aortic dissection with stent graft can be applied to different locations of the aortic dissection, from the classic descending aortic dissection to most aortic dissection and aortic aneurysm except for the aortic root. However, for the aortic root diseases (e.g. ascending aortic aneurysm or ascending aortic dissection, with aortic valve stenosis and/or insufficiency), they can be treated only by open surgery, such as Bentall surgery (Dacron vessel replacement surgery with artificial aortic valve, and re-implantation of the coronary artery into Dacron vessel), Wheat operation, and the like. These open surgical treatments need to perform cardiac arrest and extracorporeal circulation, with huge surgical trauma, high mortality and severe complications. Therefore, there is an urgent need for a medical device which allows for minimally invasive endovascular treatment for aortic root diseases.

### SUMMARY OF THE INVENTION

In one embodiment as described herein, provided herein is a stent graft for ascending aorta, for performing minimally invasive endovascular treatment for aortic root diseases, comprising a stent; a cover attachment at least partially covering the stent; a valve leaflet for reconstructing aortic valves, which is positioned at the proximal end of the stent and attached to the cover attachment and the stent. Alternatively, the stent graft has two windows aligning with two openings of the coronary artery.

The stent graft as described herein has the valve leaflet for reconstructing aortic valves, which are configured to allow blood to flow from the left ventricle to the aorta and prevent blood from flowing from the aorta back to the left ventricle. Therefore, for the aortic lesions involving aortic root, such stent graft can be implanted into aortic annulus to ascending aortic root by minimally invasive endovascular surgery to repair the ascending aorta lesions and reconstruct the aortic valves.

In another embodiment as described herein, the stent comprises a main stent for implanting to the ascending aorta and a first branch stent at the distal end of the main stent, for implanting to the branch arteries. The branch arteries comprise, but not limited to, innominate artery, left common carotid artery and left subclavian artery.

According to another embodiment as described herein, the stent graft can be restricted to an appropriate position in the ascending aorta by the first branch stent during minimally invasive intervention surgery. For example, the stent graft is restricted to a position to allow the proximal end of the stent graft to accurately anchor to the aortic annulus and allow the coronary windows of the stent graft to accurately align with the coronary openings, such that the aortic annulus and coronary artery can be accurately reconstructed, and tearing opening at the proximal end of the aortic dissection or arterial aneurysm can be isolated, thereby shrinking the false cavity of the aortic dissection or arterial aneurysm due to thrombosis for treatment of diseases. In addition, for the aortic diseases involving both branch artery and ascending aorta on the aortic arch, the stent graft as described herein can treat both branch artery and ascending aorta on the aortic arch by performing minimally invasive endovascular surgery one time. The integrated reconstruction of ascending aorta and aortic arch has high stability and low risk for lack of alignment with coronary windows and delayed endoleak due to movement of stent graft in short and long term, compared with combined stent graft.

In an alternative embodiment of the present invention, the stent graft has a generally cylindrical shape in its radially expanded state, with a length from about 35 to 155mm. The diameter of the proximal end ranges from 22 to 48mm. The diameter of the distal end ranges from 30 to 50mm. The diameter of the main stent may keep constant from the proximal end to the distal end along the axis thereof. Alternatively, the diameter of the main stent may be increased and then decreased from the proximal end to the distal end along the axis thereof. Alternatively, the main stent may have a section in trumpet-like structure at the proximal end. The maximum diameter of the main stent ranges from 28 to 50mm.

The stent graft as described herein can be anchored on an appropriate position upon implantation due to its shape in radially expanded state.

Further, or alternatively, the first branch stent has a generally cylindrical shape with the diameter of proximal end from 6 to 16mm and the diameter of distal end from 10 to 26mm, in the case that the stent graft is in radially expanded state. For example, the first branch stent has a trumpet-like structure, that is to say, the diameter of the first branch stent can be gradually increased from the proximal end to the distal end along its axis.

The configuration that the diameter of the distal end of the first branch stent is larger than that of the proximal end is beneficial to anchor the stent graft to an appropriate position in the branch artery of the aortic arch after implantation and prevent endoleaks from distal sources.

In another embodiment as described herein, the stent graft further comprises an inner stent. A part of the first branch stent and the inner stent are arranged within the main stent side by side. The distal end of the inner stent is generally in the same plane as that of the main stent. Alternatively, the distal end of the inner stent goes beyond that of the main stent. And the gap between the distal end of the main stent, the peripheral wall of the first branch stent, the peripheral wall of the inner stent and the inner wall of the main stent is sealed by a sealing element.

The configuration that the branch stents are arranged within the main stent can allow the main stent to well attach to the vessel wall and anchor to an appropriate position, which will exhibit great efficiency for the diseases involving middle region of the ascending aorta, such as aortic aneurysm.

In another embodiment as described herein, the stent further comprises a second brach stent adjacent to the first branch stent, for facing or entering into aortic arch.

During minimally invasive intervention surgery, the stent graft can be restricted to an appropriate position by using the second branch stent in addition to the first branch stent. For example, the stent graft can be firmly anchored to the ascending aorta to avoid movement or endoleak from the distal end. In addition, for the aortic diseases involving the first two or three of the coronary artery, ascending aorta, and proximal aortic arch, use of the stent graft as described herein can achieve complete endovascular cure by performing minimally invasive endovascular surgery one time. The stent graft as described herein has high stability and low risk for lack of alignment with coronary windows and delayed endoleak due to movement of stent graft in short and long term, compared with combined stent graft. Meanwhile, the second branch stent can act as a connection section for implantation of a stent graft to reconstruct the distal end of the aortic arch and the descending aorta. And the second branch stent facing or entering into the aortic arch provides a connecting section for further implantation of the stent graft to reconstruct the distal end of the aortic arch and the descending aorta.

Alternatively, in the case that the stent graft is in a radially expanded state, the second branch stent has a generally cylindrical shape with the diameter of the proximal end from 25 to 42 mm and the diameter of the distal end from 25 to 50mm. The diameter of the second branch stent is gradually increased from the proximal end to the distal end along its axis.

The configuration that the diameter of the distal end of the second branch stent is larger than that of the proximal end is beneficial to anchor to an appropriate position in the aorta upon implantation, for avoiding endoleak from distal source.

In other alternative embodiments as described herein, the two windows for aliging with two coronary arteries are provided adjacent to the proximal end of the main stent. For example, the windows are 5 to 50mm away from the proximal end of the stent. The coronary blood supply can be preserved by providing such windows.

Further, each of the horizontally opposite edges of each window is provided with a marker for alignment during implantation of the stent graft. Based on this, during implantation of the stent graft, the X-ray developed markers on the front and rear edges of the windows are overlapped at the position of the coronary artery tangent to ensure that the windows are aligned with the coronary artery openings. Otherwise, each of of the horizontally opposite sides of at least one of middle part of the main stent and the part close to the distal end of the main stent is provided with a marker for alignment during implantation of the stent graft. As such, the accuracy of alignment can be enhanced by using a plurality of markers at various positions in the stent graft.

In other alternative embodiments as described herein, in the case that the stent graft is in a radially expanded state, the stent graft has a section in drum-like shape, which is close to the proximal end. Alternatively, the distance between the proximal edge of the drum and the proximal edge of the stent ranges from 0 to 50mm, and the maximum protrusion of the drum along the radial direction of the stent is between 3 and 15mm, and the height of the drum is between 10 and 30mm. As such, sufficient space for aortic valves can be provided at the proximal end of the stent graft to prevent the aortic valves from occluding the windows of the coronary artery. In addition, such a shape is also beneficial to attach the proximal end of the stent graft to the aortic root, which facilitates reconstruction of the ascending aorta.

In other alternative embodiments as described herein, the outer surface of the proximal end of the stent graft has a trumpet-like shape, which means the diameter of the proximal end is grandually decreased. Such trumpet-like shape has a length from 0 to 40mm and a diameter at the proximal end from 22 to 48mm.

In sum, the stent graft as described herein can be used to treat aortic root disease, such as ascending aortic dissection and aortic aneurysm involving aortic sinus, coronary artery openings, aortic valves, aortic valve stenosis or insufficiency with aortic aneurysm or/and aortic dissection and the like, through minimally invasive endovascular treatment.

### BRIEF DESCRIPTION OF THE DRAWINGS

The novel features of the invention are set forth with particularity in the appended claims. A better understanding of the features and advantages of the present invention will be obtained by reference to the following detailed description that sets forth illustrative embodiments, in which the principles of the invention are utilized, and the accompanying drawings of which:
Figure 1 shows a front-view schematic diagram of the stent graft according to one embodiment as described herein.
Figure 2 shows a top-view schematic diagram of the stent graft according to one embodiment as described herein.
Figure 3 shows a bottom-view schematic diagram of the stent graft according to one embodiment as described herein.
Figure 4 illustrates that the stent graft as shown in Figure 1 is implanted into the ascending aorta vessel.
Figure 5 shows a front-view schematic diagram of the stent graft with a drum-like proximal end according to one embodiment as described herein, in which the height of the drum is marked as H.
Figure 6 shows another front-view schematic diagram of the stent graft with a drum-like proximal end as shown in Figure 5, in which the protrusion of the drum is marked as h.
Figure 7 is a perspective schematic diagram of the stent graft according to another embodiment as described herein.
Figure 8 shows a top-view schematice diagram of the stent graft as shown in Figure 7 upon cutting off the first branch stent is outside of the main stent.
Figure 9 illustrates that the stent graft as shown in Figure 7 is implanted into the ascending aorta vessel.
Figure 10 shows experimental results obtained after the stent graft as described herein is implanted into animal bodies.
Figure 11 illustrates that the stent graft with a trumpet-like proximal end according to another embodiment as described herein is implanted into the ascending aorta vessel.

### DETAILED DESCRIPTION OF THE INVENTION

Unless otherwise defined, all technical and scientific terms used herein have the same meaning as commonly understood by one of ordinary skill in the art to which this invention belongs.

The terminology used herein is for the purpose of describing particular embodiments only and is not intended to be limiting of the invention. As used herein, the singular forms "a", "an" and "the" are intended to include the plural forms as well, unless the context clearly indicates otherwise.

As used herein, the term "generally cylindrical shape" comprises cylindrical shape and the shapes similar to the cylindrical shape. As used herein, the phrase "generally in the same plane" means to describe that the objects are in the same plane or generally in the same plane.

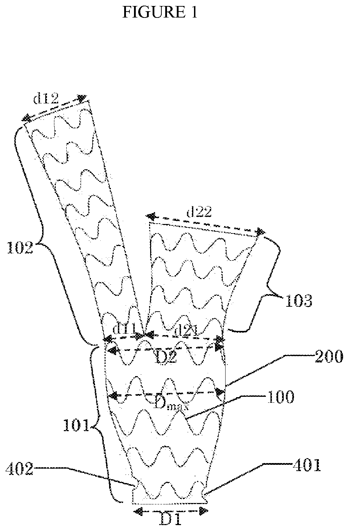

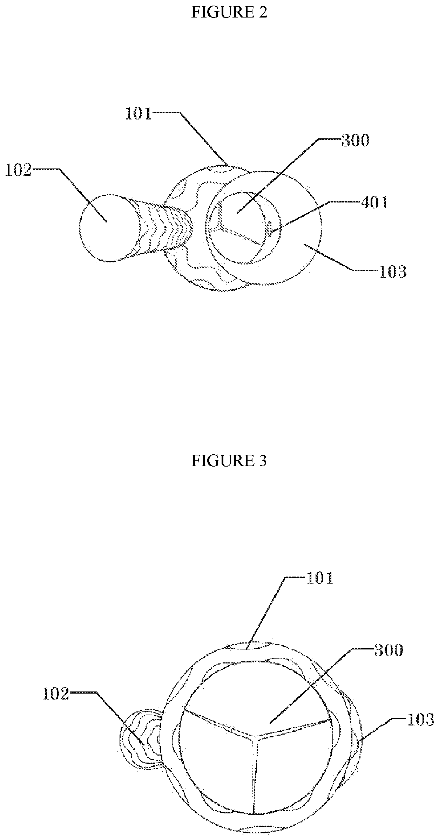

Figure 1 shows a front-view schematic diagram of the stent graft for ascending aorta according to one embodiment as described herein. Figure 2 shows a top-view schematic diagram of the stent graft according to one embodiment as described herein. Figure 3 shows a bottom-view schematic diagram of the stent graft according to one embodiment as described herein. In Figure 1, the bottom is the proximal side (end) and the top is the distal side (end). The stent graft according to one embodiment as described herein will be described as below by referring to Figures 1 to 3.

In this embodiment, the stent graft for ascending aorta can comprise: a stent 100, a cover attachment 200 covering the stent 100, a valve leaflet 300 for reconstructing aortic valves, which are positioned at the proximal end of the stent 100 and attached to the cover attachment 200 and the stent 100, and two windows 401 and 402 aligning with two coronary arteries. For example, the valve leaflet 300 may be sutured to the cover attachment 200 and the stent 100, such that the valve leaflet 300 allows blood to flow from left ventricle to aorta and prevent blood from flowing from aorta back to left ventricle when the stent graft is implanted into aorta vessel.

For ease of description, the stent 100 covered by the cover attachment 200 is shown in Figures. In this embodiment, the stent 100 acts as a supporting structure or framework of the stent graft, which may be made of stent wires, in particular, may be knitted by metal wires, or may be a self-expandable nickle-titanium stent. The cover attachment 200 can be artificial vascular membrane. The stent 100 and the cover attachment 200 can be sutured together as a whole by non-absorbable suture, or can be formed together as a whole by thermocompression or adheresion. The valve leaflet 300 can be a biological valve made of biological materials such as bovine pericardium, pig pericardium, donkey pericardium and the like. The number of the valve leaflet can be three, each of which forms a "pocket" structure by suturing to the cover attachment and the stent, thereby forming three aortic sinuses.

In this embodiment, the stent graft for ascending aorta can have two windows 401 and 402 aligning with two openings of the coronary arteries, which are close to the proximal end of the stent 100. For example, the proximal edges of the windows are 5-500mm away from the proximal end of the stent 100. For example, the proximal edges of the windows are 15mm, or 25mm, or 35mm away from the proximal end of the stent 100. The distance between the proximal edges of the windows and the proximal end of the stent 100 can be set according to the structure of the patient's arteral vessels. For example, the proximal edge of the window 401 is 25mm away from the proximal end of the stent 100 and the proximal edges of the window 402 is 27.5mm away from the proximal end of the stent 100, such that the coronary blood supply can be preserved. It should be understood that, the windows can be formed prior to surgery, or can be formed during surgery, i.e., a part of cover attachment, which aligns with the coronary arteries is removed to allow the inside of the stent to be in blood communication with the coronary arteries. In other embodiments, the cover attachment 200 can partially cover the stent 100, i.e., the area of the stent 100 which aligns with the coronary arteries is not covered by the cover attachment 200, thereby allowing the inside of the stent to be in blood communication with the coronary arteries.

In other embodiments as described herein, each of the horizontally opposite edges of each window (e.g., front and rear edges facing surgeons) is provided with a marker for alignment during implantation of the stent graft. According to this embodiment, during implantation of the stent graft, the X-ray developed markers on the front and rear edges of the windows are overlapped at the position of the coronary artery tangent to ensure that the windows are aligned with the coronary artery openings. Otherwise, each of of the horizontally opposite sides (e.g., front and rear sides facing surgeons) of at least one of middle part (e.g., the part 17-67mm away from the proximal end of the main stent) of the main stent and the part (e.g., the part 0-20mm away from the distal end of the main stent) close to the distal end of the main stent is provided with a marker for alignment during implantation of the stent graft. As such, the accuracy of alignment can be enhanced by using a plurality of markers at various positions in the stent graft. The markers can be made of any materials that are X-ray developed, such as nickle-titanium alloy and the like. And the markers can be sutured or adhered to the above positions of the stent.

In this embodiment, the stent 100 may comprise a main stent 101 for implanting into the ascending aorta and a first branch stent 102 positioned at the distal end of the main stent 101, for implanting into the branch artery, and a second branch stent 103 adjacent to the first branch stent 102 and for facing or entering into the aortic arch. The branch artery comprises any one of innominate artery, left common carotid artery, and left subclavian artery. This embodiment takes the innominate artery as an example. The skilled in the art should understand that the present invention is not limited to this and any changes can be made according to the patient's situation. This embodiment is also suitable for other branch arteries. In this embodiment, the stent graft for ascending aorta is anchored to an appropriate position of the ascending aorta by the first and the second branch stents 102 and 103, but the present invention is not limited to this. The stent graft for ascending aorta may be anchored to an appropriate position of the ascending aorta by other ways, for example, hangnails on the stent graft for locating or anchoring the stent graft. Therefore, in other embodiments, the stent 100 may merely comprise the main stent 101, or may comprise the main stent 101 and the first branch stent 102.

In this embodiment, the main stent has a length from about 35 to 155mm, for example, 57mm. The first branch stent has a length from about 45 to 105mm, for example, 80mm. The second branch stent 103 has a length from about 40 to 80mm, for example 45mm. The skilled in the art should understand that the present invention is not limited to this. The length of the main stent and the branch stents can be appropriately set according to the positions of the aorta lesion and the features of the aorta lesion. For example, the main stent 101 may have a length of 85mm. The first branch stent 102 may have a length of 45mm. The second branch stent 103 may have a length of 40mm. Or the main stent 101 may have a length of 45mm. The first branch stent 102 may have a length of 105mm. The second branch stent 103 may have a length of 80mm. The present invention will not make any specific limitation on the length of the main stent, the first branch stent and the second branch stent.

In the case that the stent graft is in radially expanded state, the main stent 101 is in cylindrical or generally cylindrical shape, whose diameter can keep constant in axial direction, or can be gradually increased and then gradually decreased. The main stent 101 may have a section of trumpet-like structure at the proximal end. For example, the diameter D1 of the main stent at the proximal end ranges from about 24 to 48mm, such as 30mm. The diameter D2 at the distal end ranges from about 30 to 50mm, such as 40mm. The maximum part has a diameter from 28 to 50mm, such as 46mm. The skilled in the art should understand that all the values as described herein are illustrative. The diameters of both ends of the main stent 101 can be set according to the patient's specific situation, which are not specifically limited herein.

In the case that the stent graft is in radially expanded state, the first branch stent 102 can be in cylindrical or generally cylindrical shape, for example, can be in trumpet-like shape. The diameter of the first branch stent 102 can be gradually increased from the proximal end to the distal end along the axial direction. In particular, the diameter d11 of the first branch stent 102 at the proximal end ranges from about 6 to 16mm, such as 10mm. The diameter d12 at the distal end ranges from about 10 to 26mm, such as 18mm. It should be noticed that, the diameters of both ends of the first branch stent 102 can be set according to the patient's specific situation, which are not specifically limited herein. The first branch stent 102 can be in any shape that allows the first branch stent 102 to attach to the vessel wall. Alternatively, in the case that the stent graft is in radially expanded state, the ratio of the diameter d11 at the proximal end of the first branch stent 102 to the diameter D2 at the distal end of the main stent 101 ranges from 1:1 to 1:6, such as 1:4. The configuration that the diameter at the distal end of the first branch stent 102 is larger than that at the proximal end is beneficial to anchor to an appropriate position in the branch artery of the aortic arch upon implantation, thereby avoiding endoleak from distal source.

In addition, in the case that the stent graft is in radially expanded state, the second branch stent 103 can be in cylindrical or generally cylindrical shape, and the diameter thereof is gradually increased from the proximal end to the distal end along the axial direction. For example, the diameter d21 at the proximal end ranges from about 25 to 42mm, such as 35mm. The diameter d22 at the distal end ranges from about 25 to 50mm, such as 40mm. The skilled in the art should understand that all the values as described herein are illustrative. The diameters of both ends of the second branch stent 103 can be set according to the patient's specific situation, which are not specifically limited herein. Alternatively, in the case that the stent graft for ascending aorta is in radially expanded state, the ratio of the diameter d21 at the proximal end of the second branch stent 103 to the diameter D2 at the distal end of the main stent 101 ranges from about 1:4 to 1:1, such as 3:4.

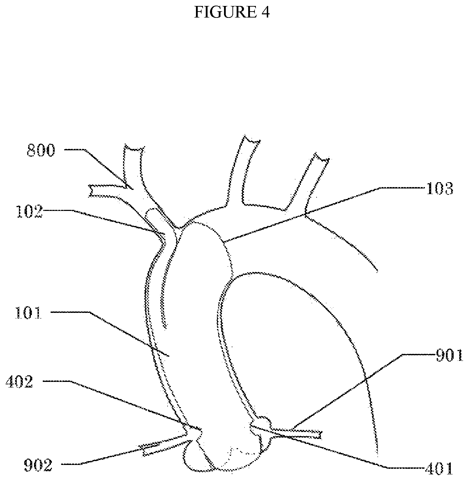

As shown in Figure 4, during minimally invasive intervention surgery, the first branch stent 102 can enter into innominate artery 800 and thus the stent graft for can be restricted to an appropriate position in the ascending aorta. For example, the proximal end of the stent graft can be accurately anchored to aortic annulus. The coronary windows 401 and 402 on the stent graft can accurately align with the openings 901 and 902 of the coronary artery. After accurately reconstructing the aortic valves and the coronary artery, the tearing opening at the proximal end of the dissection or aortic aneurysm can be isolated, so as to shrink the false cavity of the dissection or aortic aneurysm due to thrombosis. In addition, the aortic diseases involving both branch artery and ascending aorta on the aortic arch can be treated by using the stent graft as described herein through minimally invasive intervention surgery one time. Such integrated reconstruction of the ascending aorta-aortic arch has high stability and low risk for lack of alignment of coronary windows and delayed endoleak due to movement of stent graft in short and long term, compared with combined stent graft.

Further, as shown in Figure 4, the stent graft for ascending aorta can be restricted to an appropriate position of the ascending aorta by using the second branch stent 103 in addition to the first branch stent 102. For example, the stent graft can be firmly anchored to the ascending aorta to avoid displacement along the aortic axis or endoleak from distal end. In addition, for the aortic diseases involving the first two or three of the coronary artery, ascending aorta, and proximal aortic arch, use of the stent graft for the ascending aorta as described herein can achieve complete endovascular cure by performing minimally invasive endovascular surgery one time. The stent graft for the ascending aorta as described herein has high stability and low risk for lack of alignment of coronary windows and delayed endoleak due to movement of stent graft in short and long term, compared with the combined stent graft. Meanwhile, the second branch stent 103 facing or entering into the aortic arch provides a joint for further implantation of the stent graft to reconstruct the distal end of the aortic arch and the descending aorta.

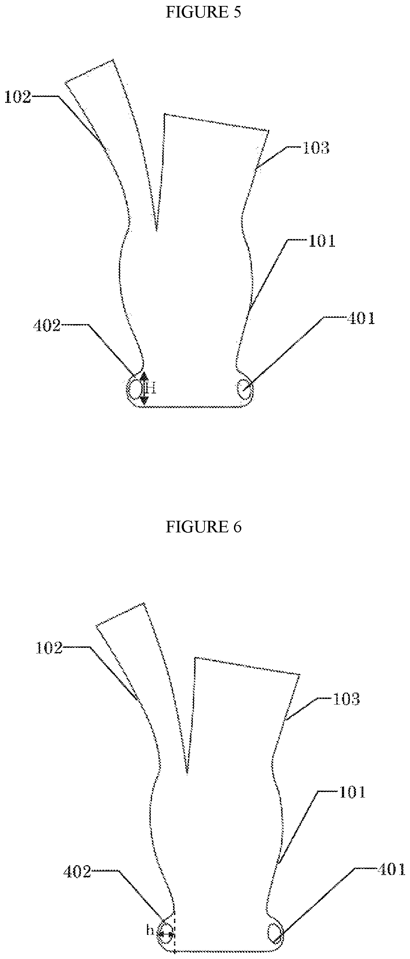

Figure 5 shows a front-view schematic diagram of the stent graft with a drum-like proximal end according to another embodiment as described herein, in which the height of the drum is marked as H. Figure 6 shows another front-view schematic diagram of the stent graft with a drum-like proximal end as shown in Figure 5, in which the protrusion of the drum is marked as h. For clarity, the stent wires and the cover attachment are not shown in Figures 5 and 6.

The stent graft as shown in Figures 5 and 6 is different from that as shown in Figures 1 to 3 in that a section of the main stent 101, which is close to the proximal end is in a drum-like shape, for matching the shape of the aortic sinus. In particular, in the case that the stent graft is in radially expanded state, the proximal edge of the drum is 0-50mm away from the proximal end of the stent graft, and the height H of the drum ranges from 10 to 30mm. The maximum protrusion h of the drum along the radial direction of the stent ranges from 3 to 15mm. That is to say, the highest point of the drum protrudes 3-15mm relative to the baseline (vertical dotted line). The specific values of H and h can be set as needed, for example, can be set according to the anatomical structure of the ascending aorta of the patient, such that sufficient space for movement of aortic valves can be provided at the proximal end of the stent graft to prevent the aortic valves from occluding the windows of the coronary artery, and the space for preserving coronary openings is provided for the purpose of endovascularly re-implanting aortic valves. In addition, such a shape is also beneficial to attach the proximal end of the stent graft to the aortic root, which enhances isolation efficiency of the stent graft for tearing opening in the aortic sinus and facilitates the reconstruction of the ascending aorta.

As shown in Figure 4, in the stent graft as described herein, the stent covered by artificial vessel membrane plays a role in isolation of aortic lesions (tearing opening in dissection or aortic aneurysm), which can be cylindrical type or branched type. The branched type stent graft has two branches at the distal end, one is a long leg (i.e., the first branch stent 102) for placing in brachiocephalic artery (i.e., innominate artery), and the other is a short leg (i.e., the second branch stent 103) facing the aortic arch, for ease of further connection of stent graft to reconstruct aortic arch. The biological aortic valves are sutured to the proximal end of the stent for reconstructing aortic valves. The windows on the artificial vessel membrane which correspond to the coronary artery are used for preserving coronary blood supply.

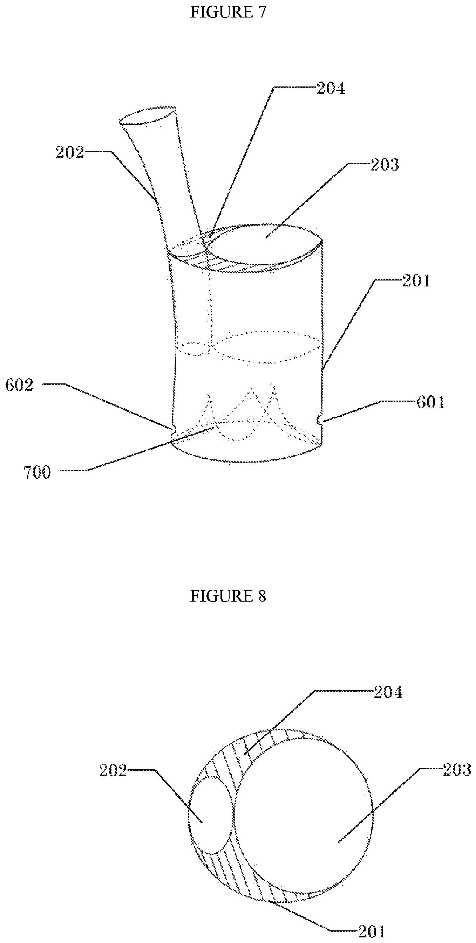

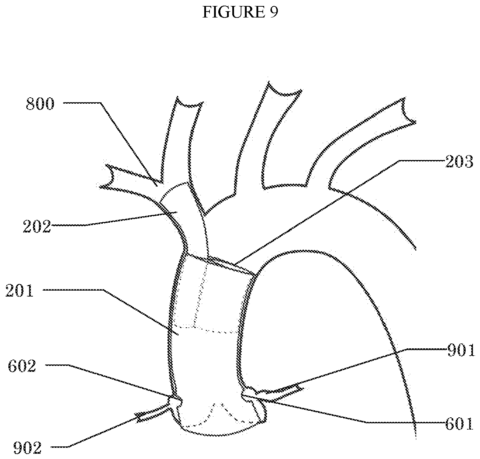

Figure 7 is a perspective schematic diagram of the stent graft according to another embodiment as described herein. Figure 8 shows a top-view schematice diagram of the stent graft as shown in Figure 7 upon cutting off the first branch stent outside of the main stent. Figure 9 illustrates that the stent graft as shown in Figure 7 is implanted into the ascending aorta vessel. For clarity, the stent wires and the cover attachment are not shown in Figures 7 to 9.

In this embodiment, as shown in Figures 7 and 8, the stent graft can comprise a main stent 201, a first branch stent 202 and an inner stent 203. The skilled in the art should understand that the stent graft further comprises artificial vessel membranes covering the stents, although they are not shown. As shown in Figure 7, a part of the first branch stent 202 and the inner stent 203 are arranged within the main stent 201 side by side. The distal end of the inner stent 203 is flush with the distal end of the main stent 201, i.e., both of them are generally in the same plane. Alternatively, the distal end of the inner stent 203 goes beyond the distal end of the main stent 201. As shown in Figure 8, the gap between the peripheral wall of the first branch stent 202, the peripheral wall of the inner stent 203 and the inner-peripheral wall of the main stent 201 is sealed by a sealing element, such that the blood cannot flow through the gap among these three stents. In addition, the valve leaflet 700 for reconstructing the aortic valves are positioned at the proximal end of the main stent 201. Two windows 601 and 602 for aligning with two coronary arteries are provided close to the proximal end of the main stent 201.

As shown in Figure 9, during minimally invasive intervention surgery, the first branch stent 202 goes into innominate artery 800. The branch stent 202 and the inner stent 203 are sleeved within the main stent 201, such that the main stent 201 can be well attached to the vessel wall, and the proximal end of the stent graft can be accurately anchored to the aortic annulus, and the coronary windows 601 and 602 on the stent graft can accurately align with the openings 901 and 902 on the coronary artery. After accurately reconstructing aortic valves and coronary arteries, the tearing opening in proximal dissection or aneurysm can be isolated, thereby shrinking the falsing cavity and aneurysm due to thrombosis. Compared with the embodiment as shown in Figure 4, since the branch stent 202 and the inner stent 203 are sleeved within the main stent 201, the main stent 201 can be well attached to the vessel wall, thereby resulting in excellent efficiency for the lesion involving the middle region of the ascending aorta, such as aneurysm.

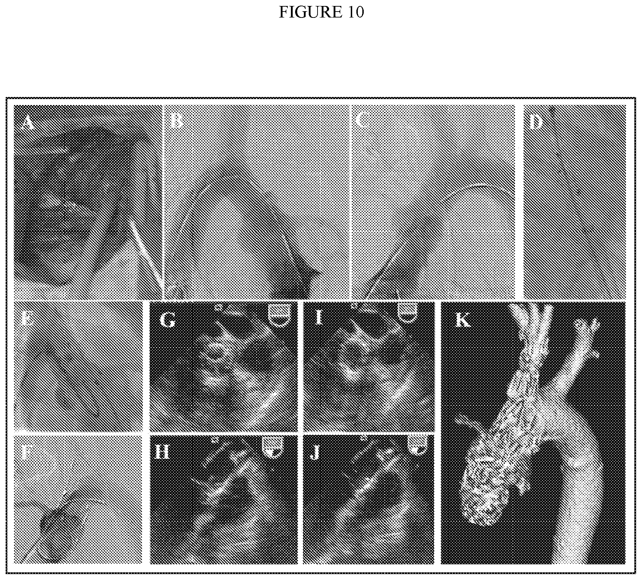

Although various embodiments of the present invention are described in greater detail by referring to the Figures, the animal experimental results of the stent graft for ascending aorta of this embodiment will be described by referring to Figure 10 for the purpose of making the features and advantages of the present invention apparent.

Healthy adult domestic pigs (60-65 kg) were used to perform animal experiments. Firstly, CT angiography (CTA) of the aorta in the domestic pigs was performed. After clarifying the anatomical structure of the aortic root, the stent graft for ascending aorta was made, comprising: biological aortic valves made of bovine pericardium and sutured to the proximal end, two windows close to the proximal end for preserving the left and right coronary artery openings. The distal end of the stent graft was branched with a long leg (i.e., the first branch stent) for placing in the brachiocephalic artery and a short leg (i.e., the second branch stent, since the pig's ascending aorta segment was very short, the length of the short leg was less than 2cm) in which only one opening facing the aortic arch was preserved. The main body of the stent graft was in a cylindrical structure. Under general anesthesia, a small incision was used to dissect the apex of the pig's heart (see Figure 10A). After confirming the angle and position of the left and right coronary artery openings by angiography (see Figures 10B and 10C), the stent graft was introduced through apex. After ensuring that the axial and circumferential position can well preserve the coronary arteries (see Figure 10D), the stent graft was released. The position of the stent graft, the function of the aortic valves and the coronary artery were evaluated by angiography in combination with esophageal ultrasonography (see Figures 10E to 10J). The aortic CTA during the follow-up period of animal experiments confirmed (see Figure 10K) that the stent graft for ascending aorta can be used to minimally invasively reconstruct the aortic root (including the aortic valves) and the ascending aorta, and keep the left and right the coronary arteries, thereby providing a joint for further reconstruction of the aortic arch and descending aorta. The stent graft for ascending aorta was suitable for treating the following diseases: ascending aortic dissection and aortic aneurysm involving aortic sinus, coronary artery openings, aortic valves, severe aortic valve insufficiency or severe stenosis with ascending aortic aneurysm, etc..

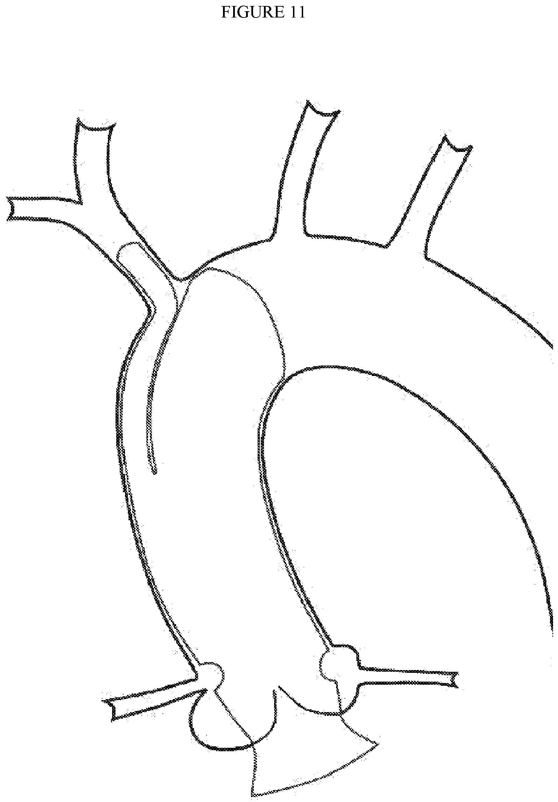

Figure 11 illustrates that the stent graft with a trumpet-like proximal end according to another embodiment as described herein is implanted into the ascending aorta vessel and left ventricular outflow tract. This embodiment is different from that as shown in Figures 1 to 4 in that a trumpet-like structure is added to the proximal end of the stent graft. That is to say, in the case that the stent graft as shown in Figure 11 is in radially expanded state, the proximal end of this stent graft is in a trumpet-like structure, which is 0-40mm away from the proximal edge of the valve leaflet, i.e., suitable for going into left ventricular outflow tract, and the diameter of the proximal end of the trumpet-like structure ranges from 22 to 48mm.

Alternatively, a trumpet-like structure can be attached to the proximal end of the stent graft of the embodiments as shown in Figures 5-6 and 7-9.

The trumpet-like structure is suitable for inserting into the left ventricular outflow tract so as to anchor the stent therein. The skilled in the art should understand that the present invention is not limited to the trumpet-like structure. Any structure whose diameter at the proximal end is large and gradually decreased can be used.

Although the preferred embodiments of the present invention have been described and illustrated herein, it is obvious to those skilled in the art that these embodiments are only for the purpose of illustration. It will be apparent to those skilled in the art that numerous variations, modifications and substitutions can be made to these embodiments without departing from the scope and spirit of the present invention. The scope of the present invention is defined by the appended claims and the methods and structures as fall within the claims together with the equivalents thereof are intended to be embraced by the appended claims.

## Claims

1. A stent graft for ascending aorta, comprising:
a stent;
a cover attachment at least partially covering the stent;
a valve leaflet for reconstructing aortic valves, which is positioned at the proximal end of the stent and attached to the cover attachment and the stent.

2. The stent graft of claim 1, wherein, the stent comprises a main stent for implanting to the ascending aorta and a first branch stent at the distal end of the main stent, for implanting to the branch arteries.

3. The stent graft of claim 2, wherein, the main stent has a length from 35 to 155mm.

4. Thestent graft of claim 2, wherein, the first branch stent has a length from 45 to 105mm.

5. The stent graft of claim 2, wherein the stent graft has a generally cylindrical shape with the diameter of proximal end from 22 to 48mm and the diameter of distal end from 30 to 50mm, in the case that the stent graft is in radially expanded state.

6. The stent graft of claim 5, wherein, the diameter of the main stent is gradually increased and then gradually decreased from the proximal end to the distal end along the axis thereof, and wherein the maximum diameter ranges from 28 to 50mm.

7. The stent graft of claim 2, wherein, the first branch stent has a generally cylindrical shape with the diameter of proximal end from 6 to 16mm and the diameter of distal end from 10 to 26mm, in the case that the stent graft is in radially expanded state.

8. The stent graft of claim 7, wherein the diameter of the first branch stent is gradually increased from the proximal end to the distal end along its axis.

9. The stent graft of claim 2, wherein the ratio of the diameter of the proximal end of the first branch stent to the diameter of the distal end of the main stent ranges from 1:1 to 1:6, in the case that the stent graft is in radially expanded state.

10. The stent graft of claim 2, wherein the stent further comprises a second branch stent adjacent to the first branch stent, for facing or entering into the aortic arch.

11. The stent graft of claim 10, wherein the second branch stent has a length from 40 to 80mm.

12. The stent graft of claim 10, wherein the second branch stent has a generally cylindrical shape with the diameter of the proximal end from 25 to 42 mm and the diameter of the distal end from 25 to 50mm, in the case that the stent graft is in a radially expanded state.

13. The stent graft of claim 12, wherein the diameter of the second branch stent is gradually increased from the proximal end to the distal end along its axis.

14. The stent graft of claim 10, wherein the ratio of the diameter of the proximal end of the second branch stent to the diameter of the distal end of the main stent ranges from 1:4 to 1:1, in the case that the stent graft is in radially expanded state.

15. The stent graft of claim 1, wherein the valve leaflet is inside the stent and sutured to the stent and the cover attachment.

16. The stent graft of claim 1, wherein two windows for aligning with two coronary arteries are provided close to the proximal end of the stent.

17. The stent graft of claim 16, wherein the proximal edge of each window is 5-50mm away from the proximal end of the stent.

18. The stent graft of claim 16, wherein r a marker for alignment during implantation of the stent graft is provided at the edge of each window, horizontally opposite to each other.

19. The stent graft of claim 18, wherein a marker for alignment during implantation of the stent graft is provided at both sides of at least one of the middle portion of the main stent and the portion close to the distal end of the main stent, horizontally opposite to each other.

20. The stent graft of claim 1, wherein the stent graft has a section in drum-like shape, which is close to the proximal end, in the case that the stent graft is in a radially expanded state.

21. The stent graft of claim 20, wherein the distance between the proximal edge of the drum-like shape and the proximal end of the stent graft ranges from 0 to 50mm.

22. The stent graft of claim 20, wherein the drum-like shape has a height from 10 to 30mm and the maximum protrusion of the drum-like shape along the radial direction of the stent ranges from 3 to 15mm, in the case that the stent graft is in a radially expanded state.

23. The stent graft of claim 2, further comprisingan inner stent;
wherein:
a part of the first branch stent and the inner stent are arranged within the main stent side by side; the distal end of the inner stent is generally in the same plane as that of the main stent; or the distal end of the inner stent goes beyond that of the main stent; and at the distal end of the main stent, the gap between the outter wall of the first branch stent, the outter wall of the inner stent and the inner wall of the main stent is sealed by a sealing element.

24. The stent graft of claim 1, wherein the outer surface of the proximal end of the stent graft has a trumpet-like shape, in the case that the stent graft is in a radially expanded state.

25. The stent graft of claim 24, wherein the distance between the proximal end of the trumpet-like shape and the proximal edge of the valve leaflet ranges from 0 to 40mm and the diameter of the proximal end of the trumpet-like shape ranges from 22 to 48mm.
